# EUROPEAN PATENT SPECIFICATION

(11) **EP 2 774 389 B1**
(45) Date of publication and mention of the grant of the patent: **11.04.2018**
(21) Application number: 12888489.7
(22) Date of filing: 16.11.2012
(51) Int. Cl.: B06B 1/06, G01N 29/28, G01N 29/24, A61B 8/12

(54) **ULTRASOUND TRANSDUCER AND MANUFACTURING METHODS THEREOF**
ULTRASCHALLWANDLER UND HERSTELLUNGSVERFAHREN DAFÜR
TRANSDUCTEUR ULTRASONORE ET SES PROCÉDÉS DE FABRICATION

(43) Date of publication of application: 10.09.2014
(73) Proprietor: Acist Medical Systems, Inc., Eden Prairie, MN 55344 (US)
(72) Inventor: BAUTISTA, Richard, Palo Alto, California 94301 (US); ZELENKA, Robert, Milipitas, California 95035 (US)
(74) Representative: Pistolesi, Roberto
(86) International application number: PCT/US2012/065706
(87) International publication number: WO 2014/077836

(56) References cited:
- EP-A1- 1 416 255
- EP-A1- 2 405 671
- EP-A2- 0 620 048
- WO-A1-2012/144117
- WO-A2-02/052544
- WO-A2-2005/104210
- US-A1- 2004 113 522
- US-A1- 2004 190 377
- US-B1- 6 278 224
- US-B1- 6 310 426
- US-B2- 7 830 069

## Description

### BACKGROUND

This application relates to ultrasonic transducers. The application further relates to ultrasonic transducers used for medical imaging.

A trade-off in medical ultrasound imaging is depth of penetration and spatial resolution. Higher ultrasound imaging frequencies enable higher spatial resolution at the expense of depth of penetration. Lower ultrasound imaging frequencies enable deeper penetration at the expense of spatial resolution. It would be useful if a single ultrasound imaging device was able to image across a broad range of frequencies in order to operate at a higher frequency for better spatial resolution and at a lower frequency for deeper penetration.

Broad bandwidth ultrasound imaging devices may include use of high sensitivity materials (e.g., single crystal piezoelectric composites), use of multiple matching layers, use of multiple transducers, and use of multiple devices. These approaches can be expensive and be difficult to implement from a manufacturing perspective, particularly for small, single-use, high-frequency ultrasound devices that are used in relatively high volumes (e.g., intravascular ultrasound catheters).

It would be advantageous to have an ultrasound transducer structure and corresponding manufacturing process that enables broadband imaging performance for small, single-use, high-frequency ultrasound devices. It would be further advantageous if the transducer is cost effective and easy to manufacture.

WO 2012/144117 discloses an ultrasonic transducer similar to the one set forth in the preamble of claim 1. Other similar transducers are disclosed in EP 0620048, WO 02/052544 and EP1416255.

### SUMMARY

An ultrasonic transducer according to the present invention includes a backing element, an active element overlying the backing element, and a matching element overlying the active element. The matching element having an inner surface that contacts the active element and an outer surface with a non-homogenous texture.

The matching element comprises a single matching layer where the outer surface has a first region with a first texture and a first material composition and a second region with a second texture and a second material composition. The first texture differs from the second texture. The first material composition may differ from the second material composition.

The first and second textures of the matching layer may be coarse or rough. The first and second regions have a reduced thickness in the matching layer. The first and second textures may be formed by ablation. The first and second textures may be formed by abrasion.

The matching element may include a plurality of matching regions having different thicknesses. The matching regions may be arranged side-by-side on the active element. At least two of the matching regions may be overlapping.

Furthermore, the matching layer may include a plurality of discrete matching regions of a first material over the active element. The matching element may further include a fill-in matching region of a second material with a different composition from the first material deposited between the discrete matching regions over the active element. The discrete matching regions of the first material and the discrete matching regions of the second material may be of the same thickness thereby forming a matching layer formed from two materials with different compositions.

In a further embodiment, a method of making an ultrasonic transducer includes the steps of providing a backing element, providing an active element overlying the backing layer, and forming a matching element over the active element, the matching element having an inner surface that contacts the active element and an outer surface with a non-homogeneous texture.

The matching element comprises a single matching layer and the forming step may include providing the outer surface with a first region having a first texture and a first material composition and a second region having a second texture and a second material composition. The first texture differs from the second texture. The first material composition may differ from the second material composition.

The matching layer has a thickness, and the step of providing the outers surface with first and second regions includes a step of reducing the thickness of the matching layer. The reducing step may include ablation. The reducing step may include abrasion.

The forming step may include providing the matching layer with a plurality of matching regions. The step of providing the matching layer with a plurality of matching regions may include arranging the matching regions side-by-side on the active element. The step of providing the matching layer with a plurality of matching regions may include overlapping at least two of the matching regions.

The forming step may include depositing a plurality of discrete matching regions of a first material on the active element. The forming step may further include forming a fill-in matching region of a second element between the discrete matching regions of the first material deposited on the active element. The method may further include the further step of causing the discrete matching regions of the first material and the discrete matching regions of the second material to have the same thickness.

### BRIEF DESCRIPTION OF THE DRAWINGS

The drawings are not to scale (unless so stated) and are intended for use in conjunction with the explanations in the following detailed description. Some embodiments will hereinafter be described in conjunction with the appended drawings, wherein like numerals denote like elements.
**FIG. 1** is a perspective view of a prior art transducer stack in accordance with an embodiment.
**FIG. 2** is a section view of a transducer stack with a matching element having two matching regions in accordance with an embodiment.
**FIG. 3** is a section view of a transducer stack with a matching element having more than two matching regions in accordance with an embodiment.
**FIG. 4** illustrates laser ablation of a matching element of a transducer stack in accordance with an embodiment.
**FIG. 5** is a perspective view of a transducer stack with a laser-ablated matching element in accordance with an embodiment.
**FIG. 5A** is a section view of the transducer stack shown in **FIG. 5****.**
**FIG. 6** illustrates micro-abrasive blasting of a matching element of a transducer stack in accordance with an embodiment.
**FIG. 7** is a perspective view of a transducer stack with a laser-ablated and micro-abrasive blasted matching element in accordance with an embodiment.
**FIG. 7A** is a section view of the transducer stack shown in **FIG. 7****.**
**FIG. 8** illustrates a time-domain response of an ultrasonic transducer stack before ablation in accordance with an embodiment.
**FIG. 9** illustrates a frequency-domain response of a transducer stack before ablation in accordance with an embodiment.
**FIG. 10** illustrates a time-domain response of a transducer stack after ablation in accordance with an embodiment.
**FIG. 11** illustrates a frequency-domain response of a transducer stack after ablation in accordance with an embodiment.
**FIG. 12** illustrates a time-domain response of a transducer stack after ablation that is excited at a first frequency in accordance with an embodiment.
**FIG. 13** illustrates a frequency-domain response of a transducer stack after ablation that is excited at a first frequency in accordance with an embodiment.
**FIG. 14** illustrates a time-domain response of a transducer stack after ablation that is excited at a second frequency in accordance with an embodiment.
**FIG. 15** illustrates a frequency-domain response of a transducer stack after ablation that is excited at a second frequency in accordance with an embodiment.
**FIG. 16** is a top view of a matching element stencil in accordance with an embodiment.
**FIG. 17** is a section view of a transducer stack having matching regions formed from a first material based on the stencil shown in **FIG. 16****.**
**FIG. 18** is a section view of a transducer stack having a second material formed over matching regions formed from a first material based on the stencil shown in **FIG. 16****.**
**FIG. 19** is a section view of a transducer stack having matching regions formed from a first material and a matching region formed from a second material based on the stencil shown in **FIG. 16****.**

### DETAILED DESCRIPTION OF THE PREFERRED EMBODIMENTS

The following description provides some practical illustrations for implementing some embodiments of the present invention. Examples of constructions, materials, dimensions, and manufacturing processes are provided for selected elements, and all other elements employ that which is known to those of ordinary skill in the field of the invention. Those skilled in the art will recognize that many of the noted examples have a variety of suitable alternatives.

For example, this application provide certain examples of a transducer stack appropriate for use in an intravascular ultrasound (IVUS) catheter having an ultrasound transducer disposed within the catheter sheath. These examples are given for illustrative purposes only and do not limit the application of the invention to only IVUS catheters.

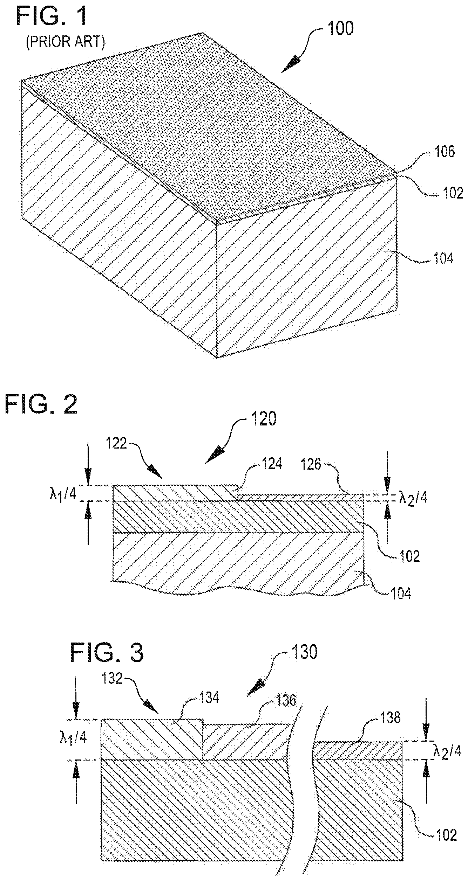

**FIG. 1** illustrates a prior art ultrasound transducer stack ***100*** having a backing layer ***104,*** an active element ***101*** that includes a single active layer ***102,*** and a matching element ***105*** that includes a single matching layer ***106.*** Transducer stack ***100*** is illustrated as having rectangular shape. In other examples, the transducer stack *100* may have other shapes, including square, circle, and oval. Transducer stack ***100*** may also include at least one electrode layer (not shown), which may be formed from metal, including gold and chrome. In one example, the transducer stack ***100*** may include two electrode layers located on the top and bottom surfaces of active element ***101,*** respectively. The electrode layer generally facilitates electrical excitation of the active layer. Transducer stack ***100*** may be electrically connected to a signal generator (not shown) to electrically excite the transducer stack. Transducer stack ***100*** may also be electrically connected to a receiver (not shown) to detect pressure fields that are converted to electrical signals by the transducer stack.

FIG. 1 shows active element ***101*** which includes active layer ***102.*** Active layer ***102*** may also be referred to as a piezoelectric layer. In other examples, active element ***101*** may include multiple active layers. Active layer ***102*** may be composed of a ceramic material such as lead zirconate titanate, commonly known as PZT. The thickness of active layer ***102*** determines the thickness resonance of the layer. For example, a 36 µm Motorola 3203HD material has a thickness resonance of approximately 63 MHz. Alternatively, active layer ***102*** may be composed of a composite material such as lead magnesium niobate-lead titanate single crystal, commonly known as PMN-PT and polymer, wherein a resonance is determined by a longitudinal length mode rather than a thickness mode.

Backing layer ***104*** may be composed of an electrically conductive epoxy, such as a tungsten-loaded epoxy. In the example of a transducer stack for use in an IVUS catheter, the thickness of backing layer ***104*** may be 200 µm or greater. In other examples, the appropriate thickness of backing layer ***104*** should be sufficiently thick to attenuate ultrasonic vibrations from active element ***101*** in the backwards direction (toward backing layer).

**FIG. 1** also illustrates matching element ***105*** which includes matching layer ***106.*** Matching layer ***106*** may be composed of an electrically conductive epoxy, such as a silver-loaded epoxy. Matching layer ***106*** provides a better acoustic impedance match between active element ***101*** and the medium in which transducer stack ***100*** is located. Matching layer ***106*** may have a uniform thickness that is equal to one quarter of the wavelength at the nominal center frequency of transducer stack ***100*** and is commonly referred to as a quarter-wave matching layer. Matching element ***105*** improves the efficiency of the transducer stack to transmit ultrasound vibrations into the surrounding medium and to receive ultrasound vibrations from the surrounding medium. While transducer stack ***100*** is shown for illustrative purposes in ***FIG.* 1** to have matching element ***105*** having only one matching layer ***106,*** in other examples, matching element *105* may have more than one matching layer to further improve efficiency.

**FIGS. 2** and **3** are section views of transducer stacks that illustrate matching elements having side-by-side matching regions. **FIG. 2** shows transducer stack ***120*** which includes backing layer ***104,*** active element *101* including active layer ***102,*** and matching element ***122.*** Matching element ***122*** includes a quarter-wave matching region ***124*** tuned to a first wavelength λ₁ with a thickness equal to λ₁/4. Matching element ***122*** also includes a quarter-wave matching region ***126*** tuned to a second wavelength λ₂ with a thickness equal to λ₂/4. Matching regions ***124, 126*** may be formed from the same material, such as a silver-loaded epoxy. In other examples, matching regions ***124, 126*** may be formed from materials with different composition. For example, matching region ***124*** may be formed from a silver-loaded epoxy having a first volume concentration of silver, while matching region ***126*** may be formed from a silver-loaded epoxy having a second volume concentration of silver. The volume concentration of silver may affect mass density and speed of sound of matching element ***122*** which in turn affect the corresponding wavelength at a given ultrasound frequency. The first and second regions of matching regions ***124, 126*** may then exhibit different corresponding quarter wavelengths. The volume concentration of silver may also affect acoustic impedance of matching element ***122.*** Matching regions ***124, 126*** may exhibit different efficiencies at different ultrasound frequencies to transmit ultrasound vibrations into the surrounding medium and to receive ultrasound vibrations from the surrounding medium.

**FIG. 3** illustrates transducer stack ***130*** having matching element ***132*** which includes a plurality of matching regions ***134-138.*** As can be appreciated, matching regions ***134-138*** of matching element ***132*** may be formed from the same material or different materials. Matching regions ***134-138*** of the matching element ***132*** may then exhibit different corresponding quarter wavelengths. The portions of transducer stack ***120*** having matching regions ***134-138*** of matching element ***132*** may exhibit different efficiencies at different ultrasound frequencies to transmit ultrasound vibrations into the surrounding medium and to receive ultrasound vibrations from the surrounding medium.

This application discloses a transducer stack having a matching layer that is matched at more than one ultrasound frequency to improve the transmit and receive efficiency of the transducer stack for a broader range of ultrasound frequencies. There are a number of techniques that may be used to form a matching element that is matched at more than one ultrasound frequency. Subtractive techniques like machining, grinding or etching may be used to modify the thickness profile of a matching layer in a matching element. Other subtractive techniques such as laser ablation or micro-abrasive blasting modify the thickness of the matching element and may also modify the composition profile of the matching element. For example, when a matching layer formed from silver-loaded epoxy is laser ablated or abrasively blasted, more of the softer epoxy may be removed compared to the silver. This would change the mass density of the ablated/blasted regions which may have an effect on the ultrasonic properties of the matching element. Generally, subtractive techniques will also increase the effective surface area of the matching element which can impact on the ultrasonic properties of the matching element.

These subtractive techniques may be used individually, or in combination to form a matching element with a coarse or roughened surface. The coarse or roughened surface of the matching element creates a varying and non-uniform thickness allowing the matching element to match to more than one ultrasound frequency. Furthermore, the coarse or roughened surface of the matching element results in an increased effective surface area of the matching element, can impact the ultrasonic properties of the matching element. Precise control of the matching element modification process will provide further improvements. An ultrasound transducer stack having a matching element with finely controlled, coarseness or roughness enables balancing the amount of transducer area matched to different ultrasound frequencies.

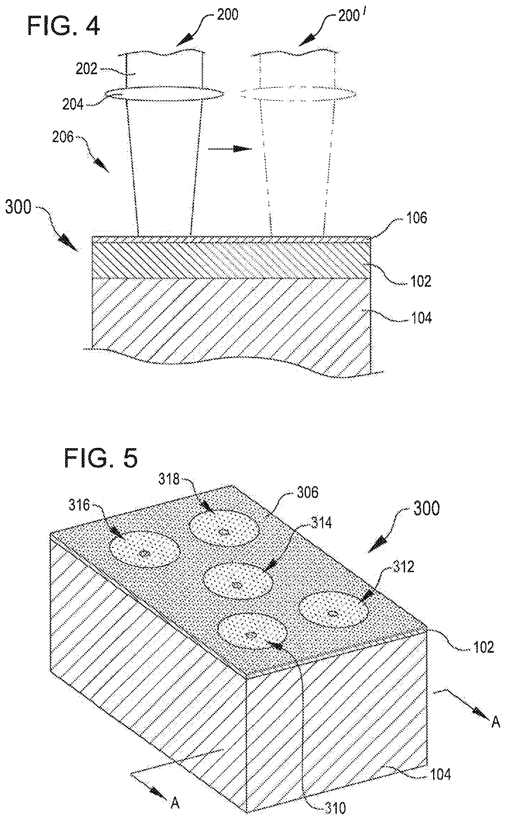

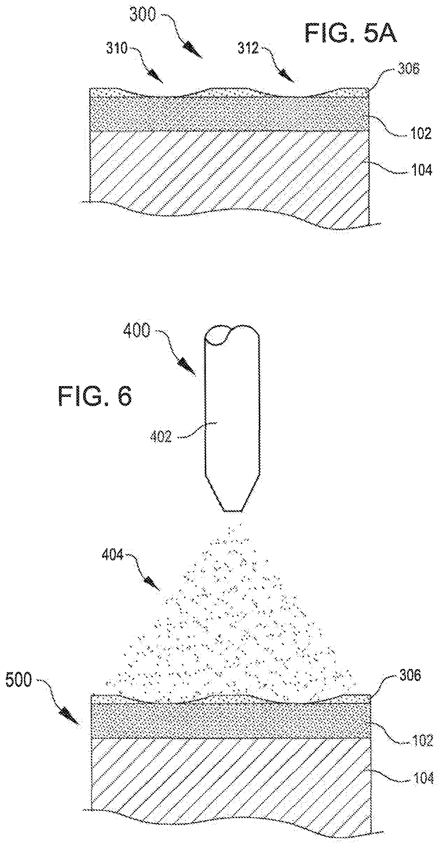

One example of a subtractive technique that may be used to form a matching element able to match at more than one ultrasound frequency is laser ablation. **FIG. 4** illustrates a laser system ***200*** ablating a surface of matching element ***105*** of transducer stack ***300.*** Laser system ***200*** includes a light source (not shown) that may operate in the near-infrared spectrum wherein the optical wavelength can vary between 800 nm and 2500 nm. Exemplary laser sources that operate in the near-infrared spectrum include neodymium-doped yttrium aluminum garnet (or ND:YAG) lasers, laser diodes, and fiber lasers. The light source generates laser beam ***202*** that may be directed through lens ***204.*** Focused laser beam ***206*** ablates a surface of matching element ***105*** to form an ablated region (see **FIG. 5**). Laser system ***200*** may be repeatedly translated to ablate multiple regions of a surface of matching element ***105.*** Alternatively, transducer stack ***300*** may be translated relative to the laser system ***200.*** The ablated region size and depth for a given laser system may be controlled by the laser system pulse energy, pulse duration and laser beam diameter.

**FIG. 5** illustrates transducer stack ***300*** having matching element ***105*** including matching layer ***306.*** Matching element ***105*** is shown to have five (5) ablated regions ***310-318.*** **FIG. 5A** shows a section view of transducer stack ***300*** including ablated regions ***310, 312.*** In the example of a transducer stack for use in an IVUS catheter, the number of ablated regions may range from one (1) to 40, wherein the diameter of the ablated regions may range from 50 µm to 500 µm. The ablated regions may be distributed uniformly or unevenly across the face of the matching layer. In other examples, the appropriate size, number and location of laser-ablated regions on the matching element may vary depending on the specific application of the transducer stack.

Another example of a subtractive technique that may be used to form a matching element able to match at more than one ultrasound frequency is micro-abrasive blasting. **FIG. 6** illustrates micro-abrasive blasting system ***400*** that is ablating matching element ***305.*** Micro-abrasive blasting system ***400*** includes abrasive nozzle ***401.*** Micro-abrasive blasting system ***400*** delivers a stream of abrasive particles ***403*** to matching element ***305*** of transducer stack ***500*** typically using a pressurized gas such as nitrogen or dry air. In the example of a transducer stack for use in an IVUS catheter, the size of the abrasive particles may range from 10 µm to 200 µm and include soft abrasives such as wheat starch or sodium bicarbonate; the depths of the ablated regions generally range between 0.1 µm and 10 µm; the pressure of the pressurized gas may range between 40 PSI and 140 PSI; and the area of the abrasive-blasted region is generally the entire surface area of the matching element. In other examples, the appropriate size and hardness of the abrasive particles, depth of ablated regions, pressure of the pressurized gas, and area of abrasive blasting may vary depending on the specific application of the transducer stack.

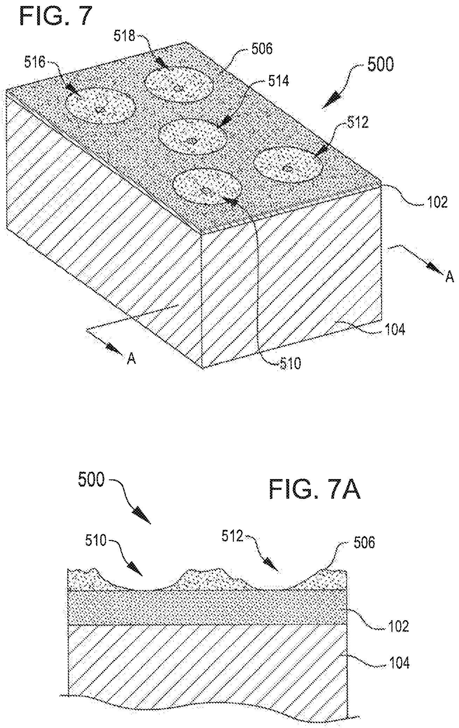

Subtractive techniques may be used in combination to further increase the transmit and receive efficiency of a transducer stack over a broader range of frequencies. **FIG. 7** illustrates transducer stack ***500*** having an abrasive-blasted and a laser-ablated matching element ***505.*** The surface of matching element ***505*** is shown to have laser-ablated regions ***510-518.*** **FIG. 7A** shows a section view of transducer stack ***500*** that includes ablated regions ***510, 512*** that have been laser-ablated and abrasive-blasted.

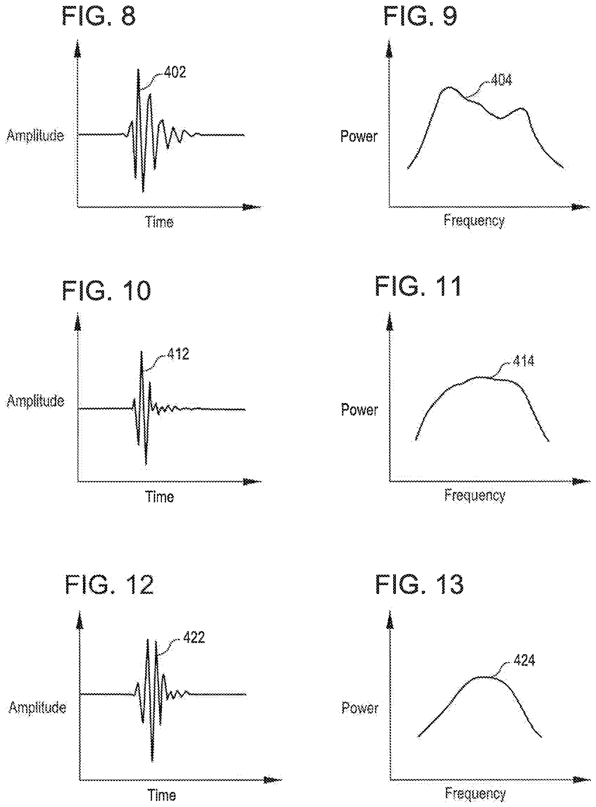

**FIGs. 8** to **11** illustrate the effect of matching layer ablation on pulse-echo time-domain and frequency-domain responses of an ultrasonic transducer stack to a short-time electrical excitation. Measurement of the pulse-echo time-domain and frequency-domain responses of an ultrasonic transducer stack are known to those skilled in the art of ultrasound imaging. **FIG. 8** shows a time-domain pulse-echo response ***402*** of the transducer stack ***300*** before ablation of the matching layer ***106,*** as illustrated in **FIG. 4****.** **FIG. 9** shows a pulse-echo (frequency-domain) power spectrum ***404*** that corresponds to the time-domain pulse-echo response ***402*** of the transducer stack ***300*** before ablation of a matching layer ***106.*** **FIG. 10** shows a time-domain pulse-echo response ***412*** of the transducer stack ***500*** after laser ablation and abrasive blasting of the matching layer ***506,*** as illustrated in **FIG. 7A****.** **FIG. 11** shows a pulse-echo (frequency-domain) power spectrum ***414*** that corresponds to the time-domain pulse-echo response ***412*** of the transducer stack ***500*** after laser ablation and abrasive blasting of the matching layer ***506.*** The effects of matching element ablation on pulse-echo time-domain and frequency-domain responses of the ultrasonic transducer stack are decreased time-domain pulse length, increased center frequency, and increased bandwidth. These effects generally provide improved image quality of ultrasound devices.

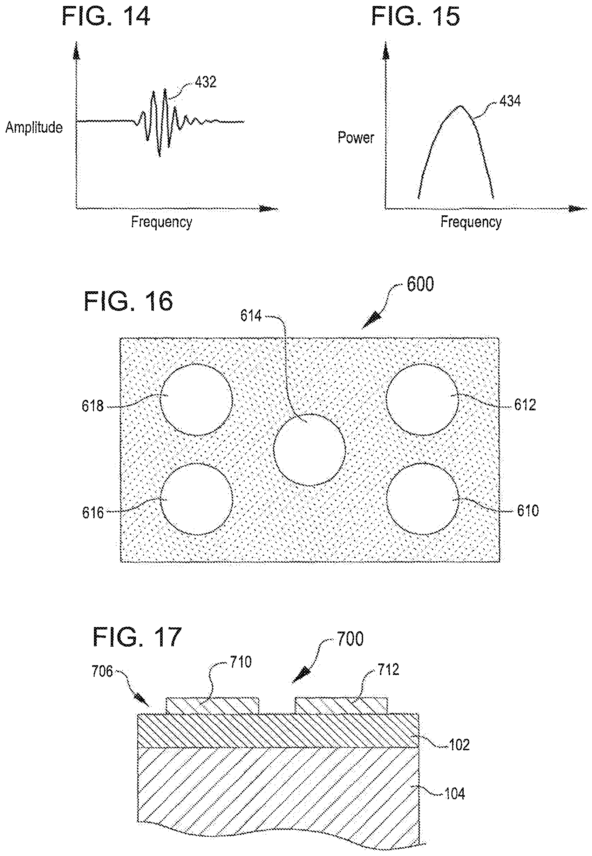

The increased bandwidth further enables imaging at more than one frequency. **FIGs. 12** to **15** show the pulse-echo time-domain and frequency-domain responses of transducer stack ***500*** having an ablated matching element ***506,*** as illustrated in **FIG. 7****.** **FIGs. 12** and **13** respectively show a pulse-echo time-domain response ***422*** and a pulse-echo (frequency-domain) power spectrum ***424*** of a short-time electrical excitation having a first frequency. **FIGs. 14** and **15** respectively show a pulse-echo time-domain response ***432*** and a pulse-echo (frequency-domain) power spectrum ***434*** of a short-time electrical excitation having a second frequency, wherein the second frequency is lower than the first frequency. The pulse-echo time-domain response ***422*** of the transducer to the first-frequency, short-time, electrical excitation is shorter than the pulse-echo time-domain response ***432*** of the transducer to the second-frequency, short-time, electrical excitation. The pulse-echo power spectrum ***424*** of the transducer to the first-frequency, short-time, electrical excitation has a higher center frequency than that of the pulse-echo power spectrum ***434*** of the transducer to the second-frequency, short-time, electrical excitation. A transducer operating with a shorter time-domain pulse and higher center frequency will generally enable imaging with better spatial resolution and a smaller depth of penetration. Conversely, a transducer operating with a longer time-domain pulse and lower center frequency will generally enable imaging with a larger depth of penetration and lower spatial resolution.

Deposition techniques may also be used to increase the transmit and receive efficiency of a transducer stack over a broader range of frequencies. In one technique, one or more stencils may be used to form a matching layer of a matching element, the matching layer having multiple matching regions formed from materials with different compositions. Stencils can be developed from metals, such as stainless steel. Stencil patterns can be fabricated using known processes, such as photochemical machining. A stencil includes at least one cut-out hole that may be of a variety of shapes, including circle, rectangle, or triangle. In the example of a transducer stack for use in an IVUS catheter having a width of approximately 0.5 mm and a length of approximately 0.75 mm, the thickness of the stencil may range from 0.05 mm to 1 mm, and the cut-out holes may vary in size from approximately 0.025 mm to 0.5mm. In other examples, the dimensions of the stencil and the size and shape of the cut-out-holes may vary depending on the specific application of the transducer stack.

**FIG. 16** illustrates a top view of an example of a stencil ***600*** that may be used to deposit a first material on a transducer stack that may be used in an IVUS catheter. The stencil length is approximately 0.75 mm, width is approximately 0.5 mm, and thickness is approximately 0.05 mm. The stencil ***600*** includes five (5) cut-out holes ***610-618*** wherein the cut-holes are circular in shape and have diameters of approximately 0.15 mm.

**FIG. 17** shows a section view of transducer stack ***700*** including matching element ***705*** having partial matching layer ***706.*** Partial matching layer ***706*** includes matching regions ***710, 712*** formed from a first material. Matching regions ***710, 712*** are formed by aligning stencil ***600,*** shown in **FIG. 16****,** with the top surface of transducer stack ***700.*** The first material, such as an epoxy containing a first volume fraction of silver, is then applied to transducer stack ***700.*** Excess first material may be removed by scraping the top surface of stencil ***600*** using a razor blade or other sharp-bladed instrument after the first material is applied. Stencil ***600*** may then be removed from the top surface of transducer stack ***700*** thereby forming matching regions **710, 712** formed from the first material. Matching regions **710, 712** may then be permitted to cure before depositing additional materials.

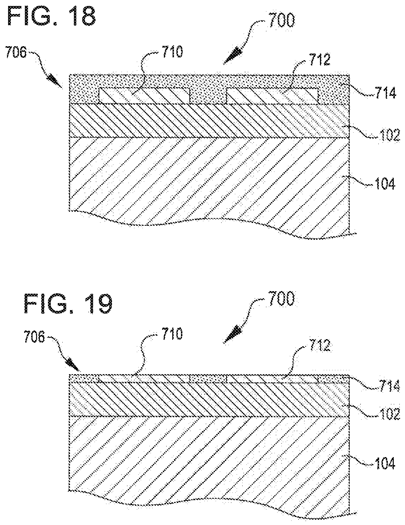

**FIG. 18** shows a section view of transducer stack **700** after having a second material ***714,*** having a different composition from the first material, deposited on the top face of transducer stack ***700*** that include matching regions ***710, 712*** formed from the first material. The second material ***714*** may then be permitted to cure before applying a subtractive technique to reduce the thickness of matching layer ***706*** to a target thickness. The thickness of matching layer ***706*** can be reduced by a variety of techniques, for example machining. ***FIG. 19*** shows a section view of the transducer stack ***700*** having a matching element ***705*** with a matching layer ***706,*** the matching layer ***706*** including matching regions ***710, 712*** formed from the first material and matching region ***714*** formed from the second material. It can be appreciated that in other examples, a matching element may include a matching layer formed from more than two materials, each material having a different composition.

This application discloses a number of subtractive and deposition techniques, each method may be used individually to increase the transmit and receive efficiency of a transducer stack over a broad range of frequencies. It can be appreciated, that any of these methods may also be used in combination with each other to further increase the efficiency of a transducer stack. For example, as noted above, **FIG. 7** shows a transducer stack ***500*** having a matching element having been both laser-ablated and abrasive-blasted. In another example, the transducer stack ***700,*** as shown in **FIG. 19****,** may have its matching element ***705*** coarsened or roughened using either laser ablation, abrasive blasting, or both. In yet another example, these techniques may be performed on transducer stack ***120*** and ***130*** as shown in **FIGs. 2** and **3****,** respectively.

Furthermore, the subtractive and deposition techniques disclosed in this application may be used individually or in combination on varying transducer stacks. For example, these techniques may be performed on transducer stack ***100*** shown in **FIG. 1** which includes backing layer ***104,*** active element ***101*** having a single active layer ***102,*** and matching element ***105*** having a single matching layer ***106.*** In another example, these techniques may be performed on a transducer stack including a backing layer, an active element having one or more active layers, and a matching element having one or more matching layers. In yet another example, using **FIG. 19** as a reference, these techniques may be applied to a transducer stack ***700*** having an active element ***101*** with more than one active layer, and a matching element ***705*** with more than one matching layer, where one of those layers is similar to matching layer ***706.***

In the present invention, an ultrasonic transducer is provided. The transducer includes an active element having a first side and a second side. The transducer includes a backing element attached to the first side of the active element. The transducer includes a matching element attached to the second side of the active element. The matching element has a surface that is coarse or roughened causing the matching element to have a non-uniform thickness.

Such an ultrasonic transducer includes a variety of characteristics. The coarse or roughened surface of the matching element includes a plurality of concavities. The concavities may be ablated regions. In some embodiments, the ablated regions may have diameters ranging between 50 µm and 500 µm. In some embodiments, by use of micro-abrasive blasting, the ablated regions may cover up to the entire surface of the transducer. In some embodiments, the matching element may include at least two matching layers. In some embodiments, the active element may further comprise two active layers.

In the present invention, an ultrasonic transducer is provided. The transducer can include an active element having a first side and a second side. The transducer can include a backing element attached to the first side of the active element. The transducer further includes a matching element attached to the second side of the active element. The matching element includes at least one matching layer. At least one of the matching layers includes at least a first matching region formed from a first material and a second matching region formed from a second material. The first and the second materials can be formed from materials having different compositions.

Such an ultrasonic transducer can include a variety of characteristics. In the embodiments, a surface of the matching element may be coarse or roughened. The matching element has a non-uniform thickness. The coarse or rough surface includes a plurality of concavities. In such embodiments, the concavities may be ablated regions. In some embodiments, the ablated regions may have diameters ranging between 50 µm and 500 µm. In some embodiments, by use of micro-abrasive blasting, the ablated regions may cover up to the entire surface of the transducer. In some embodiments, the matching element may include at least two matching layers. In some embodiments, the active layer may include at least two active layers.

Some embodiments provide a method of manufacturing an ultrasonic transducer. These embodiments involve providing an active element having a first side and a second side. These embodiments involve providing a backing element on the first side of the active element. These embodiments involve forming a matching element on the second side of the active element. These embodiments include forming a surface of the matching element such that the surface is coarse or roughened. In such embodiments, the matching element has a non-uniform thickness.

Such a method to form an ultrasonic transducer includes a variety of steps. In some embodiments, at least one subtractive technique may be used to form the matching element. In some embodiments, the at least one subtractive technique may include laser ablation. In some embodiments, the at least one subtractive technique may include micro-abrasive blasting. In some embodiments, the at least one subtractive technique may include both laser ablation and micro-abrasive blasting. In some embodiments, the at least one subtractive technique may include machining, grinding, or etching. In some embodiments, forming the active element may include forming at least two active layers. In some embodiments, forming the matching element may include forming at least two matching layers.

In some embodiments, a method of manufacturing an ultrasonic transducer is provided. The embodiments involve providing an active element having a first side and a second side. The embodiments involve providing a backing element on the first side of the active element. The embodiments involve forming a matching element on the second side of the active element. In such embodiments, the matching element includes a first matching layer. In such embodiments, the first matching layer may include a first matching region formed from a first material, and a second matching region formed from a second material having a different composition than the first material.

Such a method to form an ultrasonic transducer can include a variety of steps. In some embodiments, a first deposition technique may be used in forming the first matching layer. In such embodiments, the first deposition technique may include aligning a first stencil adjacent to the second side of the active element. In such embodiments, the stencil may have at least one cut-out-hole. In some embodiments, a first material may be applied to the first stencil. In some embodiments, the first stencil is removed and the first material is allowed to cure. In such embodiments, the cured first material forms the first matching region. In some embodiments, the first deposition technique may be repeated for a second stencil. In some embodiments, a second deposition technique may be used in forming the first matching layer. In such embodiments, the second deposition technique may include applying a second material to a surface of the matching element and allowing the second material to cure. In such embodiments, the cured second material forms the second matching region. In some embodiments, a first subtractive technique is used in forming the first matching layer. In such embodiments, the first subtractive technique may include reducing the thickness of the first matching layer until the thickness of the first and second matching regions are equal. In some embodiments, the first subtractive technique may include machining, grinding, or etching. In some embodiments, a second subtractive technique is used to form the matching element. In such embodiments, the matching element may have a surface that is coarse or rough. In such embodiments, the matching element may have a non-uniform thickness. In some embodiments, the second subtractive technique may include laser ablation. In some embodiments, the second subtractive technique may include micro-abrasive blasting. In some embodiments, the second subtractive technique may include both laser ablation and micro-abrasive blasting.

Thus, embodiments of the invention are disclosed.

## Claims

1. An ultrasonic transducer comprising:
a backing layer (104);
an active element (101) overlying the backing layer (104); and
a matching element (505) overlying the active element (101), the matching element (505) having an inner surface that contacts the active element (101) and an outer surface with a non-homogeneous texture wherein the matching element (505) comprises a single matching layer, the outer surface having (a) a first region (510) with a first texture and a first material composition, and (b) a second region (512) with a second texture and a second material composition, wherein the first texture differs from the second texture and
**characterised in that** the first and second regions (510, 512) have a reduced thickness in the matching layer.

2. The transducer of claim 1, wherein the first material composition differs from the second material composition.

3. The transducer of claim 1, wherein the matching element (505) includes a plurality of matching regions having different thicknesses.

4. The transducer of claim 3, wherein the matching regions are arranged side-by-side on the active element (101).

5. The transducer of claim 2, wherein the matching element (505) further includes a plurality of discrete matching regions of the first material composition over the active element (101).

6. The transducer of claim 5, wherein the matching element (505) further includes a fill-in matching region of the second material composition deposited between the discrete matching regions of the first material over the active element (101).

7. A method of making an ultrasonic transducer comprising:
providing a backing layer (104);
providing an active element (101) overlying the backing layer (104); and
forming a matching element (505) over the active element (101), the matching element (505) having an inner surface that contacts the active element (101) and an outer surface opposite the inner surface, the outer surface having a non-homogeneous texture wherein the matching element (505) comprises a single matching layer, the outer surface having (a) a first region (510) with a first texture and a first material composition and (b) a second region (512) with a second texture and a second material composition, wherein the first texture differs from the second texture and
**characterised in that** the first and second regions (510, 512) have a reduced thickness in the matching layer.

8. The method of claim 7, wherein the first material composition differs from the second material composition.

9. The method of claim 7, wherein the step of providing the outer surface with first and second regions (510, 512) includes a step of reducing a thickness of the matching layer.

10. The method of claim 9, wherein the reducing step includes ablation.

11. The method of claim 9, wherein the reducing step includes abrasion.

## Patentansprüche

1. Ultraschall-Transducer, welcher umfasst:
eine Stützschicht (104);
ein aktives Element (101), das über der Stützschicht (104) liegt; und
ein Abgleich-Element (505), das über dem aktiven Element (101) liegt, worin das Abgleich-Element(505) eine innere Oberfläche aufweist, die mit dem aktiven Element (101) in Kontakt steht, und eine äußere Oberfläche mit einer nicht homogenen Textur, worin das Abgleich-Element (505) eine Einzel-Abgleich-Schicht umfasst, die äußere Oberfläche (a) einen ersten Bereich (510) mit einer ersten Textur und einer ersten Material-Zusammensetzung aufweist, und (b) einen zweiten Bereich (512) mit einer zweiten Textur und einer zweiten Materialzusammenetzung, worin die erste Textur verschieden ist von der zweiten Textur und **dadurch gekennzeichnet, dass** der erste und zweite Bereich (510, 512) eine verringerten Dicke in der Abgleich-Schicht aufweisen.

2. Transducer nach Anspruch 1, worin die erste Materialzusammensetzung verschieden ist von der zweiten Materialzusammensetzung.

3. Transducer nach Anspruch 1, worin das Abgleich-Element (505) mehrere Abgleich-Bereiche mit unterschiedlicher Dicke umfasst.

4. Transducer nach Anspruch 3, worin die Abgleich-Bereiche an dem aktiven Element (101) Seite an Seite angeordnet sind.

5. Transducer nach Anspruch 2, worin das Abgleich-Element (505) weiter mehrere diskrete Abgleich-Bereiche der ersten Materialzusammensetzung über dem aktiven Element (101) umfasst.

6. Transducer nach Anspruch 5, worin das Abgleich-Element (505) weiter einen Auffüll-Abgleich-Bereich der zweiten Materialzusammensetzung aufweist, der zwischen den diskreten Abgleich-Bereichen des ersten Materials über dem aktiven Element (101) vorgesehen ist.

7. Verfahren zur Herstellung eines Ultraschall-Transducers, welches umfasst:
Bereitstellen einer Stütz-Schicht (104);
Bereitstellen eines aktiven Elements (101), das über der Stützschicht liegt (104); und
Ausbilden eines Abgleich-Elements (505) über dem aktiven Element (101), worin das Abgleich-Element(505) eine innere Oberfläche aufweist, die mit dem aktiven Element (101) in Kontakt steht, und eine äußere Oberfläche abgewandt von der inneren Oberfläche, worin die äußere Oberfläche eine nicht-homogene Textur aufweist, worin das Abgleich-Element (505) eine Einzel-Abgleich-Schicht umfasst, die äußere Oberfläche (a) einen ersten Bereich (510) mit einer ersten Textur und einer ersten Materialzusammensetzung aufweist und (b) einen zweiten Bereich (512) mit einer zweiten Textur und einer zweiten Materialzusammensetzung, worin die erste Textur verschieden ist von der zweiten Textur und **dadurch gekennzeichnet, dass**
der erste und zweite Bereich (510, 512) eine verringerte Dicke in der Abgleich-Schicht aufweisen.

8. Verfahren nach Anspruch 7, worin die erste Materialzusammensetzung verschieden ist von der zweiten Materialzusammensetzung.

9. Verfahren nach Anspruch 7, worin der Schritt der Bereitstellung der äußeren Oberfläche mit dem ersten und zweiten Bereich (510, 512) einen Schritt der Verringerung der Dicke der Abgleich-Schicht beinhaltet.

10. Verfahren nach Anspruch 9, worin der Verringerungsschritt eine Abtragung beinhaltet.

11. Verfahren nach Anspruch 9, worin der Verringerungsschritt Abrasion beinhaltet.

## Revendications

1. Transducteur ultrasonore comprenant :
une couche de support (104) ;
un élément actif (101) recouvrant la couche de support (104) ; et
un élément d'appariement (505) recouvrant l'élément actif (101), l'élément d'appariement (505) ayant une surface interne qui entre en contact avec l'élément actif (101) et une surface externe avec une texture non homogène,
l'élément d'appariement (505) comprenant une simple couche d'appariement, la surface externe comprenant (a) une première région (510) avec une première texture et une première composition de matériau et (b) une deuxième région (512) avec une deuxième texture et une deuxième composition de matériau, la première texture étant différente de la deuxième texture et
**caractérisé en ce que**
la première et la deuxième régions (510, 512) présentent une épaisseur réduite dans la couche d'appariement.

2. Transducteur selon la revendication 1, dans lequel la première composition de matériau diffère de la deuxième composition de matériau.

3. Transducteur selon la revendication 1, dans lequel l'élément d'appariement (505) comprend une pluralité de régions d'appariement ayant différentes épaisseurs.

4. Transducteur selon la revendication 3, dans lequel les régions d'appariement sont disposées côte à côte sur l'élément actif (101).

5. Transducteur selon la revendication 2, dans lequel l'élément d'appariement (505) comprend en outre une pluralité de régions d'appariement discrètes de la première composition de matériau au-dessus de l'élément actif (101).

6. Transducteur selon la revendication 5, dans lequel l'élément d'appariement (505) comprend en outre une région d'appariement de remplissage de la deuxième composition de matériau déposée entre les régions d'appariement discrètes du premier matériau au-dessus de l'élément actif (101).

7. Procédé de fabrication d'un transducteur ultrasonore comprenant :
la disposition d'une couche de support (104) ;
la disposition d'un élément actif (101) recouvrant la couche de support (104) ; et
la formation d'un élément d'appariement (505) au-dessus de l'élément actif (101), l'élément d'appariement (505) comprenant une surface interne qui entre en contact avec l'élément actif (101) et une surface externe opposée à la surface interne , la surface externe ayant une texture non homogène,
l'élément d'appariement (505) comprenant une simple couche d'appariement, la surface externe ayant (a) une première région (510) avec une première texture et une première composition de matériau et (b) une deuxième région (512) avec une deuxième texture et une deuxième composition de matériau, la première texture étant différente de la deuxième texture et
**caractérisé en ce que**
la première et la deuxième régions (510, 512) présentent une épaisseur réduite dans la couche d'appariement.

8. Procédé selon la revendication 7, dans lequel la première composition de matériau diffère de la deuxième composition de matériau.

9. Procédé selon la revendication 7, dans lequel l'étape de disposition de la surface externe avec une première et une deuxième régions (510, 512) comprend une étape de réduction d'une épaisseur de la couche d'appariement.

10. Procédé selon la revendication 9, dans lequel l'étape de réduction comprend une ablation.

11. Procédé selon la revendication 9, dans lequel l'étape de réduction comprend une abrasion.
